# EUROPEAN PATENT SPECIFICATION

(11) **EP 3 998 574 B1**
(45) Date of publication and mention of the grant of the patent: **13.09.2023**
(21) Application number: 20840244.6
(22) Date of filing: 22.05.2020
(51) Int. Cl.: G06T 1/00, A61B 5/1172, A61B 5/00, A61B 5/11, G06V 40/12, F21V 8/00, G06V 40/13

(54) **ELECTRONIC DEVICE**
ELEKTRONISCHE VORRICHTUNG
DISPOSITIF ÉLECTRONIQUE

(30) Priority: 12.07.2019 JP 2019129763
(43) Date of publication of application: 18.05.2022
(73) Proprietor: Sony Group Corporation, Tokyo 108-0075 (JP)
(72) Inventor: AMANO, Toru, Tokyo 108-0075 (JP); TAKAHASHI, Teppei, Fujisawa-shi, Kanagawa 251-0042 (JP); ENOKIDO, Toshio, Tokyo 108-0075 (JP)
(74) Representative: MFG Patentanwälte Meyer-Wildhagen Meggle-Freund Gerhard PartG mbB
(86) International application number: PCT/JP2020/020226
(87) International publication number: WO 2021/010017

(56) References cited:
- WO-A1-2008/123584
- CN-A- 104 794 466
- CN-A- 104 850 839
- JP-A- 2008 018 135
- JP-A- 2014 137 641
- US-A1- 2016 041 663
- US-A1- 2017 337 412
- US-A1- 2018 129 798
- US-A1- 2018 144 113
- US-A1- 2018 204 039

## Description

### TECHNICAL FIELD

The present disclosure relates to an electronic device.

### BACKGROUND ART

A device that authenticates a part of a living body (e.g., fingerprint) is known. For example, Patent Document 1 below describes a device that images a fingerprint while irradiating the fingerprint with light emitted from a light source, and performs authentication on the basis of the captured image. In Patent Document 2, a light source, such as an array of LEDs, emits light through a surface in contact with a body part so as to be reflected and partially absorbed by the body part, and an array of photodetectors detects light reflected back by the body part and generates signals corresponding to an image of the light reflection, which corresponds to the light absorption pattern in the body part. The light absorption pattern may correlate to a fingerprint, a blood vessel pattern, blood movement within the blood vessels, or other biometric feature.

### CITATION LIST

### PATENT DOCUMENT

Patent Document 1: Japanese Patent Application Laid-Open No. 2017-196319
Patent Document 2: US 2017/337412 A1

### SUMMARY OF THE INVENTION

### PROBLEMS TO BE SOLVED BY THE INVENTION

In such a field, there is a need to irradiate a part of a target living body with light efficiently.

One object of the present disclosure is to provide an electronic device that irradiates a part of a living body with light efficiently.

### SOLUTIONS TO PROBLEMS

The invention is set out in the appended set of claims.

### BRIEF DESCRIPTION OF DRAWINGS

Figs. 1A and 1B are diagrams referred to when a problem to be considered in embodiments is described.
Figs. 2A and 2B are diagrams referred to when the problem to be considered in the embodiments is described.
Fig. 3 is a diagram referred to when an appearance example of a wristband-type electronic device according to a first embodiment is described.
Fig. 4 is a block diagram illustrating a circuit configuration example of the wristband-type electronic device according to the first embodiment.
Fig. 5 is a diagram referred to when an internal structure example of the wristband-type electronic device according to the first embodiment is described.
Fig. 6 is a diagram referred to when an internal structure example of the wristband-type electronic device according to the first embodiment is described.
Fig. 7 is a diagram referred to when an internal structure example of the wristband-type electronic device according to the first embodiment is described.
Fig. 8A is a diagram illustrating an internal configuration of a part of the wristband-type electronic device according to the first embodiment taken out, and Fig. 8B is an exploded perspective view of the internal configuration.
Figs. 9A and 9B are diagrams illustrating a configuration example of a refractive part according to the first embodiment, and Fig. 9C is a diagram for describing a relative positional relationship between the refractive part and LEDs.
Fig. 10 is a diagram referred to when a specific example of the size of the refractive part according to the first embodiment is described.
Fig. 11 is a diagram referred to when an operation example of the wristband-type electronic device according to the first embodiment is described.
Figs. 12A and 12B are diagrams referred to when a prism-shaped part according to a second embodiment is described.
Figs. 13A and 13B are diagrams referred to when a specific example of the prism-shaped part according to the second embodiment is described.
Figs. 14A and 14B are diagrams referred to when another specific example of the prism-shaped part according to the second embodiment is described.
Fig. 15 is a diagram for describing a specific example of a crest-trough shape included in the prism-shaped part according to the second embodiment.
Fig. 16 is a diagram used for comparison when describing the effect obtained by forming the prism-shaped part according to the second embodiment.
Fig. 17 is a diagram referred to when describing the effect obtained by forming the prism-shaped part according to the second embodiment.
Figs. 18A and 18B are diagrams referred to when an example of a manufacturing method of the prism-shaped part according to the second embodiment is described.
Fig. 19 is a diagram referred to when an example of manufacturing method of the prism-shaped part according to the second embodiment is described.
Fig. 20 is a diagram referred to when another example of the manufacturing method of the prism-shaped part according to the second embodiment is described.
Fig. 21 is a diagram for describing a modification.
Fig. 22 is a diagram for describing a modification.
Fig. 23 is a diagram for describing a modification.
Figs. 24A to 24C are diagrams for describing a modification.

### MODE FOR CARRYING OUT THE INVENTION

Hereinafter, embodiments and the like of the present disclosure will be described with reference to the drawings. Note that the description will be given in the following order.

### <Problems to be considered in embodiments>

### <First embodiment>

### <Second embodiment>

### <Modification>

Note that the dimensions, materials, and shapes of the components described in the embodiments, the relative arrangement thereof, the description of directions such as upper, lower, left, and right directions, and the like, are not intended to limit the scope of the present disclosure unless otherwise specified, and are merely examples for description. Note that sizes, positional relationships, and the like of members illustrated in the drawings may be exaggerated for clarity of description, and a configuration may be only partially illustrated or only some of reference numerals may be illustrated for brevity of the illustration. Moreover, in the following description, the same names and reference numerals indicate the same or similar members, and redundant description will be appropriately omitted. Moreover, regarding each element included in the present disclosure, a plurality of the elements may include the same member and one member may serves as the plurality of the elements, or conversely, a function of one member may be shared and implemented by a plurality of members.

In the present embodiment, as an electronic device, an authentication device that images a fingerprint, which is one example of a part of a living body, and performs authentication using a captured fingerprint image will be described as an example. Specifically, in the present embodiment, a wearable device that is a relatively small device detachable from a human body will be described as an example of the authentication device. More specifically, a wristband-type (wristwatch-type) wearable device (hereinafter appropriately referred to as wristband-type electronic device) will be described as an example. The electronic device of the present disclosure is not limited to the wearable device, as a matter of course. The electronic device of the present disclosure may be a device incorporated in a personal computer or a smartphone.

### <Problems to be considered in embodiments>

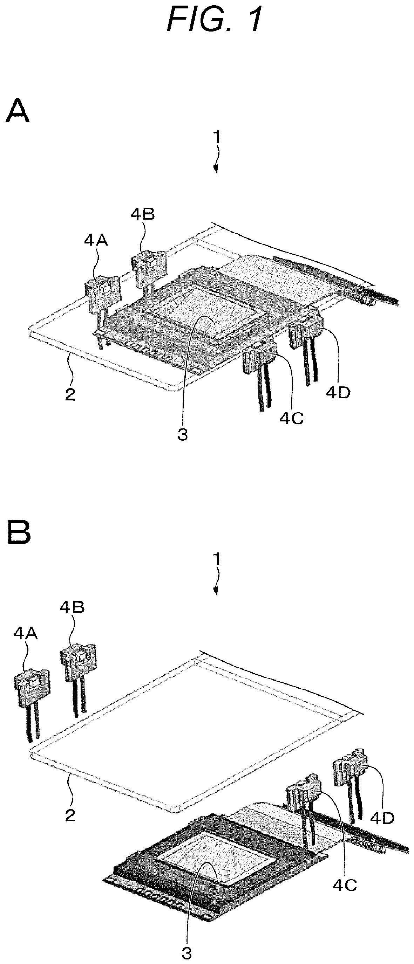

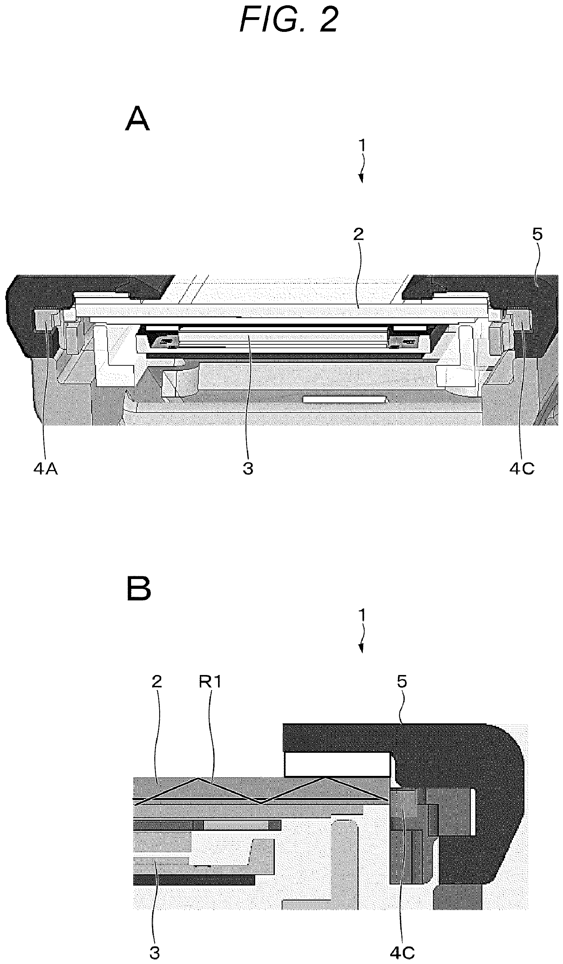

First, in order to facilitate understanding of the present disclosure, problems to be considered in the embodiments will be described with reference to Figs. 1 and 2.

Fig. 1A is a perspective view illustrating a partial configuration of a general wristband-type electronic device (hereinafter appropriately referred to as wristband-type electronic device 1), and Fig. 1B is an exploded perspective view of the part of the configuration. Additionally, Figs. 2A and 2B are a diagram of or a diagram including a cross section of the part illustrated in Figs. 1A and 1B.

The wristband-type electronic device 1 has a light guide plate 2, an image sensor 3 disposed so as to face the light guide plate 2, and four light emitting diodes (LEDs) 4A to 4D. In the light guide plate 2, a contact region is set where the fingertip is brought into contact with the vicinity of a part immediately above the image sensor 3. The fingertip brought into contact with the contact region is irradiated with light emitted from the LEDs 4A to 4D. As schematically illustrated by a light beam R1 in Fig. 2B, for example, light emitted from the LED 4C is guided to the contact region by the light guide plate 2. Then, imaging by the image sensor 3 is performed. User authentication or the like is performed by applying known processing to a fingerprint image acquired by imaging.

Meanwhile, in a general wristband-type electronic device 1, as illustrated in Figs. 1A and 1B, the LEDs 4A to 4D are disposed on side surfaces (side surfaces in longitudinal direction in present example) of the light guide plate 2. In the case of such a configuration, it is necessary to cover the vicinity of upper parts of the LEDs 4A to 4D with a cover member 5 as illustrated in Figs. 2A and 2B, so that light emitted from the LEDs 4A to 4D does not leak to the outside of the housing. Since the cover member 5 is required, an increase in the number of parts causes an increase in cost and restriction in design. Additionally, since the LEDs 4A to 4D are disposed on the side surfaces of the thin light guide plate 2, there is a problem that it is difficult to align the LEDs. A first embodiment of the present disclosure will be described in detail in consideration of such points.

### <First embodiment>

### [Wristband-type electronic device]

### (Appearance example of wristband-type electronic device)

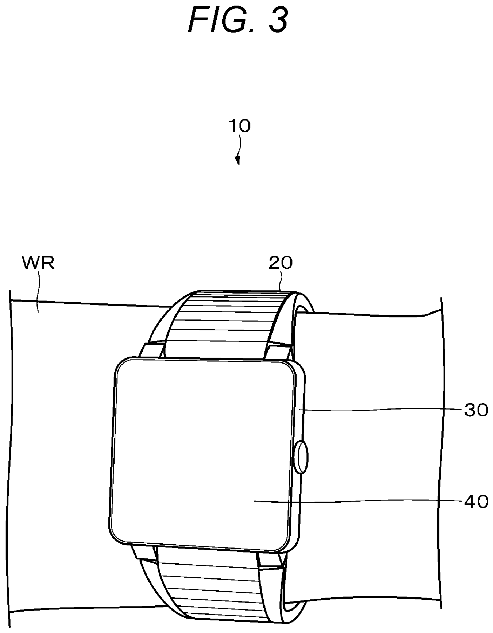

Fig. 3 is a diagram for describing an appearance example of a wristband-type electronic device (hereinafter appropriately referred to as wristband-type electronic device 10) according to the first embodiment. The wristband-type electronic device 10 has a band part 20 that is wound around a wrist WR of the user, and a body part 30. The body part 30 has a display 40. A contact region into which the fingertip is brought into contact is set on the display 40. When the user brings the fingertip into contact with the contact region, a fingerprint of the fingertip is imaged, and biometric authentication or the like using the fingerprint image can be performed. Note that imaging may be performed in a state where the fingertip is brought close to the display 40 (non-contact).

The constituent (material) included in the band part 20 may be a metal such as aluminum or stainless steel (which may be subjected to surface treatment such as gold plating), or may be skin, wood, mineral (stone), fiber (cloth), bamboo, ceramic, or a combination of any of these. The constituent included in the band part 20 may be an optically transparent member or an opaque member.

The display 40 includes a liquid crystal display (LCD), an organic light emitting diode (OLED), and the like. The constituent itself included in the band part 20 may function as a display.

### (Circuit configuration example of wristband-type electronic device)

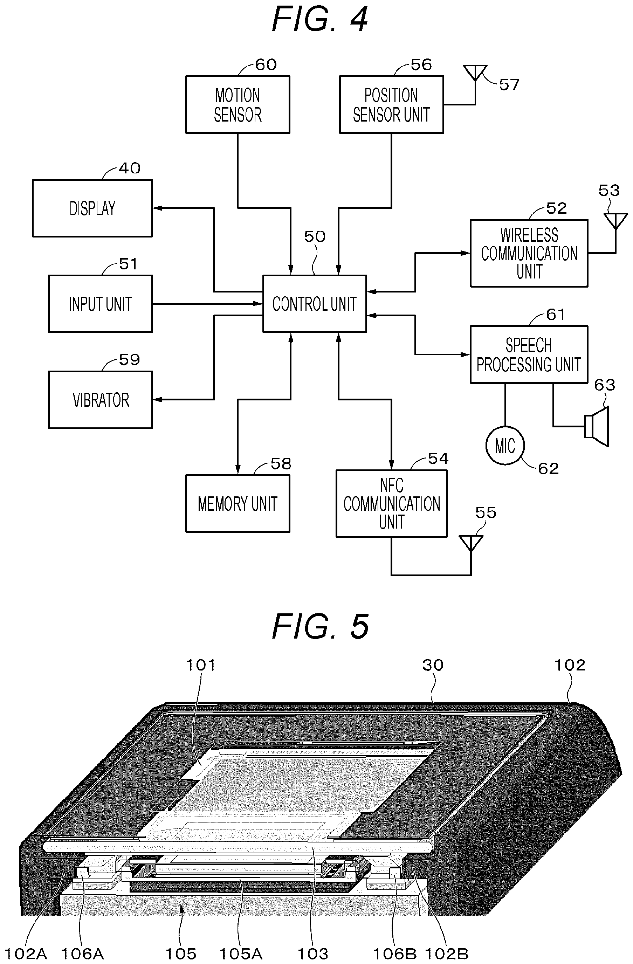

Fig. 4 is a block diagram illustrating a circuit configuration example of the wristband-type electronic device 10 according to the first embodiment. The wristband-type electronic device 10 has, in addition to the display 40 described above, a control unit 50, an input unit 51, a wireless communication unit 52, an antenna 53 connected to the wireless communication unit 52, a near field communication (NFC) communication unit 54, an antenna 55 connected to the NFC communication unit 54, a position sensor unit 56, an antenna 57 connected to the position sensor unit 56, a memory unit 58, a vibrator 59, a motion sensor 60, a speech processing unit 61, a microphone 62, and a speaker 63, for example.

The control unit 50 includes a central processing unit (CPU), for example, and has centralized control over the units of the wristband-type electronic device 10. Additionally, the control unit 50 performs known authentication processing according to fingerprint authentication.

The input unit 51 is a generic term for configurations for accepting an operation input included in the wristband-type electronic device 10. Examples of the input unit 51 include a touch panel, a button, a dial, and the like. Note that the input unit 51 may be a configuration for accepting a voice input for performing voice recognition (e.g., speaker 63).

The wireless communication unit 52 performs short-range wireless communication with another terminal on the basis of the Bluetooth (registered trademark) standard, for example. The wireless communication unit 52 performs modulation/demodulation processing, error correction processing, and the like in accordance with the Bluetooth (registered trademark) standard, for example.

The NFC communication unit 54 performs wireless communication with a nearby reader/writer on the basis of the NFC standard. Note that although illustration is omitted, power is supplied to each unit of the wristband-type electronic device 10 from a battery such as a lithium ion secondary battery. The battery may be wirelessly charged according to the NFC standard.

The position sensor unit 56 is a positioning unit that measures the current position using a system called global navigation satellite system (GNSS), for example. Data obtained by the wireless communication unit 52, the NFC communication unit 54, and the position sensor unit 56 is supplied to the control unit 50. Then, the control unit 50 performs control based on the supplied data.

The memory unit 58 is a generic term for a read only memory (ROM) in which a program executed by the control unit 50 is stored, a random access memory (RAM) used as a work memory when the control unit 50 executes the program, a nonvolatile memory for data storage, and the like.

The vibrator 59 is a member that vibrates the whole wristband-type electronic device 10, for example. Vibration by the vibrator 59 notifies the user of an incoming call, reception of an e-mail, and the like.

The motion sensor 60 detects a movement of the user wearing the wristband-type electronic device 10. An acceleration sensor, a gyro sensor, an electronic compass, a barometric pressure sensor, or the like is used as the motion sensor 60. Note that the wristband-type electronic device 10 may incorporate a sensor other than the motion sensor 60. For example, a biosensor that detects biological information other than the fingerprint such as blood pressure, pulse, sweat glands (position of sweat glands or degree of sweating from sweat glands), body temperature, or the like of a user wearing the wristband-type electronic device 10 may be incorporated. Alternatively, a pressure sensor or the like for detecting whether or not the user has worn the wristband-type electronic device 10 may be provided on the back of the band part 20.

The microphone 62 and the speaker 63 are connected to the speech processing unit 61, and the speech processing unit 61 performs call processing with a party on the other end connected by wireless communication performed by the wireless communication unit 52. Additionally, the speech processing unit 61 can also perform processing for a speech input operation.

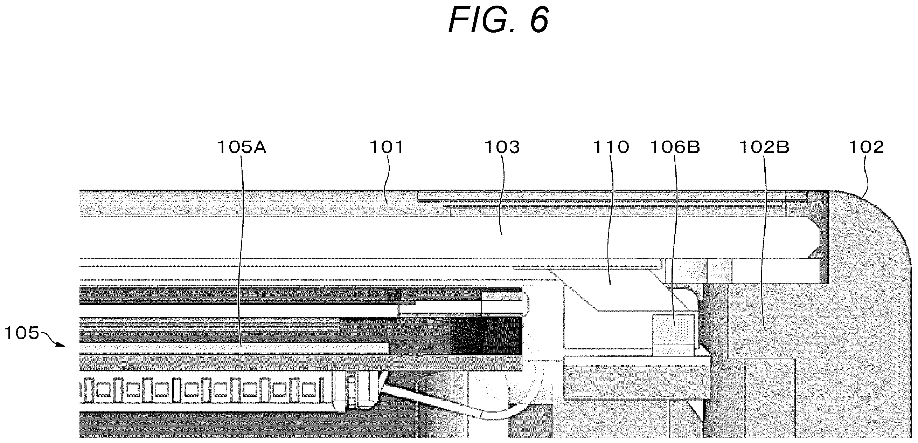

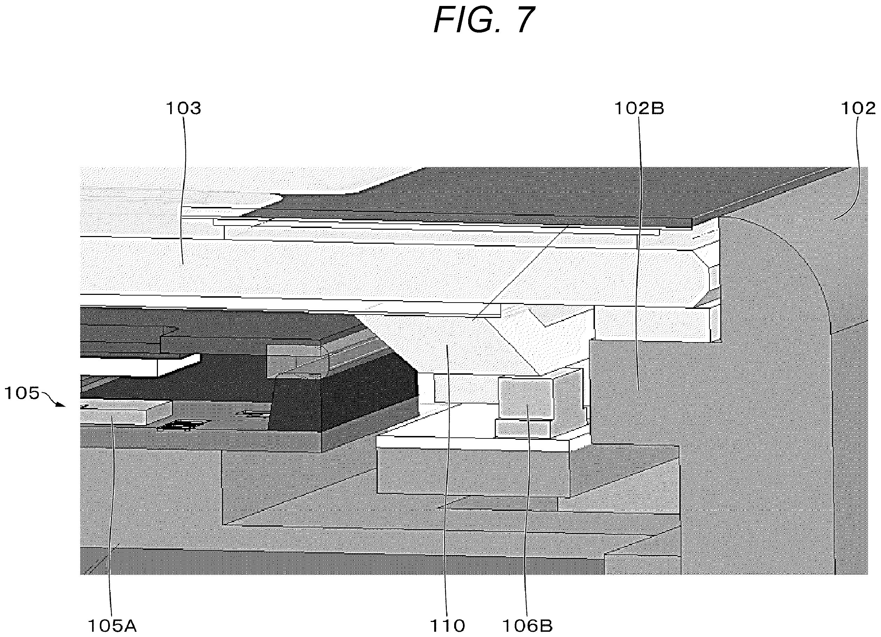

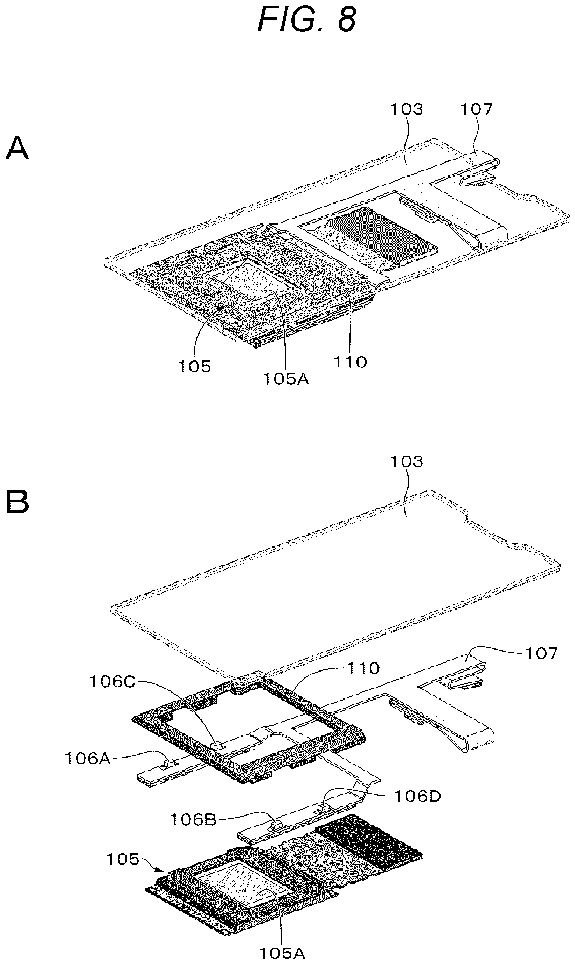

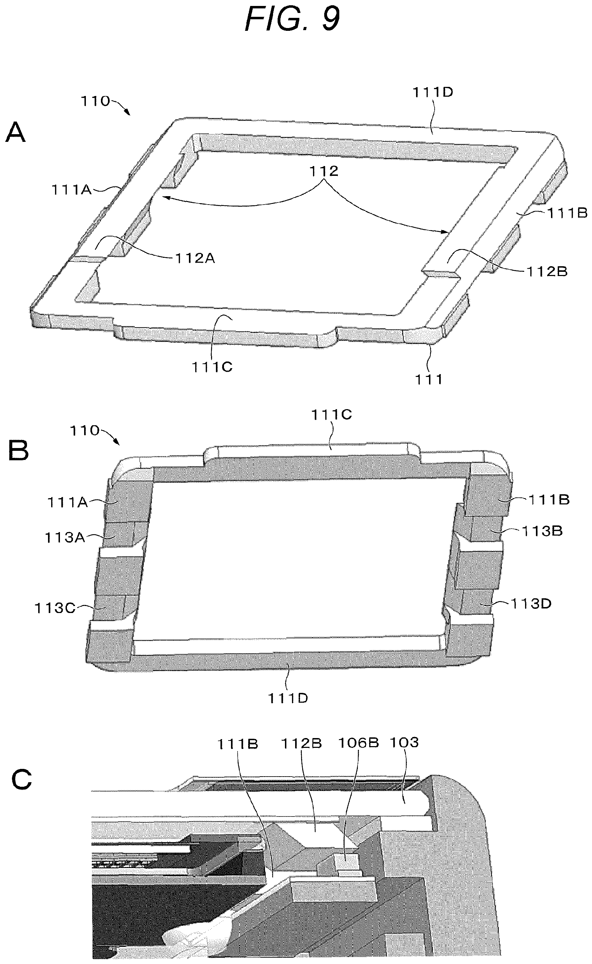

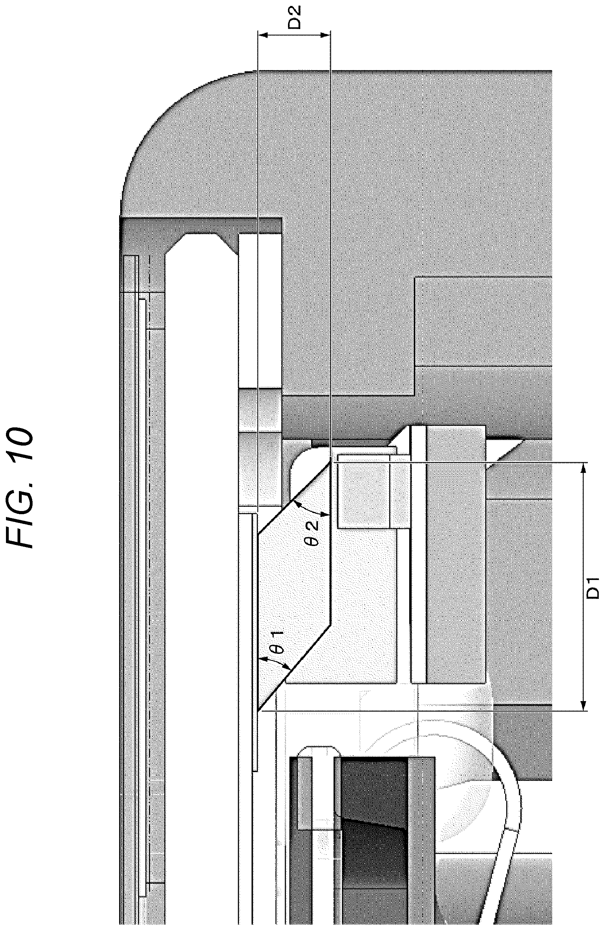

(Internal structure example of wristband-type electronic device) Next, an internal structure example of the wristband-type electronic device 10 according to the first embodiment will be described with reference to Figs. 5 to 10. Figs. 5 to 7 are diagrams referred to when an internal structure example of the wristband-type electronic device 10 (internal structure example of body part 30) is described. Fig. 8A is a diagram illustrating an internal configuration of a part of the wristband-type electronic device 10 taken out, and Fig. 8B is an exploded perspective view of the internal configuration. Figs. 9A and 9B are diagrams referred to when a configuration example of a refractive part according to the first embodiment is described, and Fig. 9C is a diagram (diagram illustrating cross section) referred to when the relative positional relationship between the refractive part and LEDs is described. Fig. 10 is a diagram referred to when a specific example of the shape of the refractive part according to the first embodiment is described.

As illustrated in Fig. 5, a cover glass 101 including an optically transparent member such as glass or acrylic is provided in the position of the display 40 of the wristband-type electronic device 10. The cover glass 101 is attached to an outer frame part 102 of the wristband-type electronic device 10 by an appropriate method. The outer frame part 102 includes metal, resin, or the like. The outer frame part 102 has a pair of protrusions 102A and 102B protruding inside the body part 30.

A rectangular light guide plate 103 is disposed inside the cover glass 101. A resin such as acrylic, urethane rubber, silicone rubber, polyurethane, polycarbonate, or cycloolefin-based resin, or various optical materials such as glass can be applied as the light guide plate 103. The light guide plate 103 is supported such that the vicinity of the periphery of its sides facing each other is placed on the protrusions 102A and 102B.

In the internal space of the body part 30, an imaging unit 105 having an image sensor 105A is disposed so as to face the light guide plate 103. Note that in the following description, a surface of the light guide plate 103 on the cover glass 101 side is appropriately referred to as a front surface, and the opposite surface (surface facing image sensor 105A) of the light guide plate 103 is appropriately referred to as a facing surface. The image sensor 105A has a rectangular shape, for example. A complementary metal oxide semiconductor (CMOS) or a charge coupled device (CCD) may be used as the image sensor 105A. A fingerprint image is acquired through the image sensor 105A.

Note that the imaging unit 105 may include, for example, an optical system including a micro lens array (MLA). Additionally, a light shielding body for controlling the directivity of the scattered light incident on the imaging unit 105 may be further provided above the micro lens array. The micro lens array includes a plurality of micro lenses that are light receiving lenses. The micro lenses are arranged in a lattice pattern on a predetermined substrate. The number and arrangement positions of the micro lenses are not particularly limited, and can be appropriately set according to the size of the living body to be imaged or the size of the image sensor 105A. Each micro lens guides the scattered light incident on the micro lens to the image sensor 105A. The micro lens array is a lens array having a small field curvature and no distortion in the depth direction. Hence, by using such a micro lens array, a favorable captured image can be obtained.

A plurality of light emitting elements is disposed around the imaging unit 105. In the present embodiment, an LED is used as the light emitting element. Additionally, in the present embodiment, four LEDs 106A, 106B, 106C, and 106D are used. Note that in a case where there is no need to distinguish the individual LEDs, the LEDs are appropriately referred to as LED 106.

The LED 106 may emit irradiation light of a single wavelength or may emit irradiation light of a plurality of wavelengths such as a full-color LED. Additionally, a small laser may be used as the various light sources. The LED 106 is mounted on a flexible printed circuit (FPC) 107 (see Fig. 8B).

Inside the body part 30, a refractive part 110 is provided which is interposed at least between the LED 106 and the light guide plate 103 and refracts the light emitted from the LED 106 to guide the light to the contact region of the fingerprint. The refractive part 110 includes a microprism, for example. The refractive part 110 is disposed around the imaging unit 105 (specifically, image sensor 105A) (see Figs. 8A and 8B).

As illustrated in Figs. 9A and 9B, the refractive part 110 includes a frame-shaped first refractive part 111. Note that in the following description, a main surface of the first refractive part 111 on the light guide plate 103 side is referred to as a front surface, and an opposite main surface of the first refractive part 111 is referred to as a back surface.

The first refractive part 111 has a first side 111A and a second side 111B facing each other. Additionally, the first refractive part 111 has a third side 111C and a fourth side 111D facing each other.

A second refractive part 112 is formed in the first refractive part 111. In the present embodiment, the first refractive part 111 and the second refractive part 112 are integrally formed. Note that "integrally formed" includes integrally molding the first refractive part 111 and the second refractive part 112 in one process, and separately forming the first refractive part 111 and the second refractive part 112 and integrating them by adhesion or the like.

The second refractive part 112 includes a second refractive part 112A and a second refractive part 112B, for example. The second refractive part 112A is formed in the vicinity of the center of the front surface of the first side 111A, for example. The second refractive part 112B is formed in the vicinity of the center of the front surface of the second side 111B, for example. The second refractive part 112A and the second refractive part 112B have a parallelogram cross-sectional shape in the transverse direction, for example.

One example of the size of the refractive part 110 will be described with reference to Fig. 10. A width D1 of the cross section of the second side 111B is set in a range of about 1.5 mm to 2.0 mm, for example. Additionally, a height D2 of the second refractive part 112A and the second refractive part 112B is set to about 0.3 mm to 0.8 mm. Additionally, the magnitudes of a pair of diagonal angles θ1 and θ2 in the cross section of the second refractive part 112A and the second refractive part 112B are set in a range of 40 degrees (°) to 45 degrees.

As illustrated in Fig. 9B, a cutout 113A and a cutout 113C are formed on the back surface side of the first side 111A. For example, the cutout 113A and the cutout 113C are formed in a position opposite to the position where the second refractive part 112A is formed. Additionally, a cutout 113B and a cutout 113D are formed on the back surface side of the second side 111B. For example, the cutout 113B and the cutout 113D are formed in a position opposite to the position where the second refractive part 112B is formed. Note that in a case where there is no need to distinguish the individual cutouts, the cutouts are collectively referred to as cutout 113 as appropriate.

The cutout 113 is a position where the LED 106 is disposed. For example, the LED 106A is disposed in the cutout 113A, the LED 106B is disposed in the cutout 113B, the LED 106C is disposed in the cutout 113C, and the LED 106D is disposed in the cutout 113D. With such an arrangement, for example, the LED 105B is disposed below the second refractive part 112B (see Fig. 9C).

### [Operation example of wristband-type electronic device]

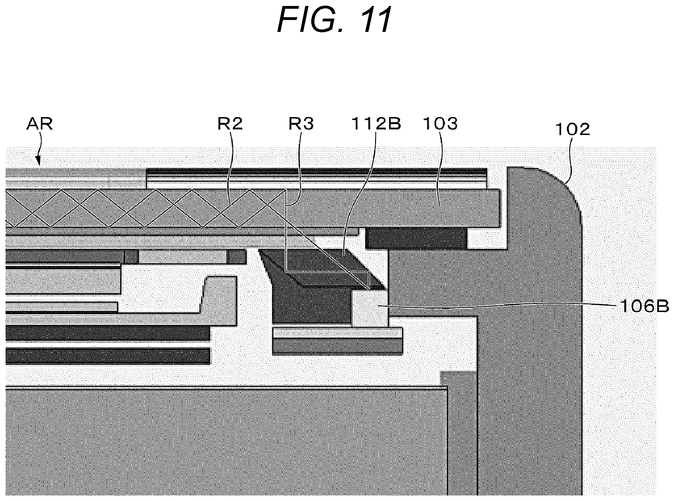

Next, an operation example of the wristband-type electronic device 10, specifically, behavior of light emitted from the LED 106 will be described with reference to Fig. 11. A light beam R2 of the light emitted from the LED 106B is guided to the light guide plate 103 through the second refractive part 112B, for example. Additionally, a light beam R3 (upward light) of the light emitted from the LED 106B is reflected by one inclined part of the second refractive part 112B to be refracted inward, and is further reflected by the other inclined part of the second refractive part 112B to be refracted upward to be guided to the light guide plate 103. The light guided by the light guide plate 103 is guided to a contact region AR while being totally reflected in the light guide plate 103. Note that light emitted from other LEDs is similarly guided to the contact region AR.

### [Effects of present embodiment]

According to the present embodiment, the light emitting element is disposed around the imaging unit. Accordingly, as compared with a mode in which the light emitting element is disposed on a side surface of the light guide plate, the light emitting element is arranged more freely.

Additionally, the light from the light emitting element can be guided to the light guide plate efficiently by the refractive part including the first refractive part and the second refractive part. Accordingly, there is no need to provide a cover or the like for preventing light leakage, and it is possible to achieve reduction in the number of parts and cost reduction associated therewith.

Additionally, since there is no need to provide a cover or the like, a waterproof structure or the like can be designed easily.

Additionally, since the light from the light emitting element can be guided to the light guide plate efficiently, a high-quality fingerprint image or the like can be obtained.

### <Second embodiment>

Next, a second embodiment will be described. Note that in the description of the second embodiment, the same or similar configurations as those in the above description are denoted by the same reference numerals, and redundant description is appropriately omitted. Additionally, matters described in the first embodiment can be applied to the second embodiment unless otherwise specified.

The second embodiment is different from the first embodiment in that a prism-shaped part is formed on a facing surface of a light guide plate 103. Note that a prism-shaped part means a set of prism shapes.

### [Shape example and arrangement example of prism-shaped part]

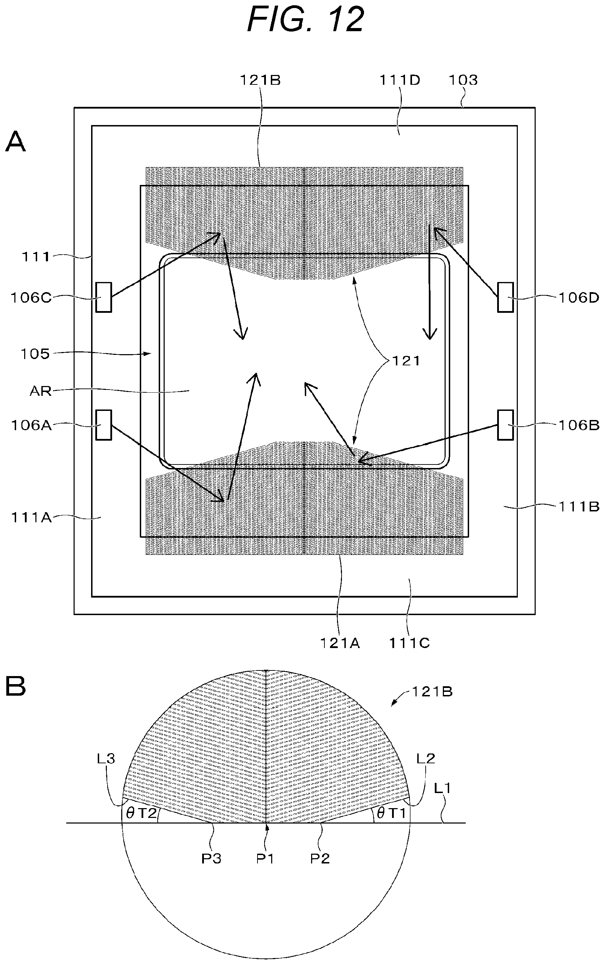

Fig. 12 is a diagram of a wristband-type electronic device 10 viewed from the upper side of the light guide plate 103. Note that in Fig. 12, illustration of some configurations (e.g., second refractive part 112) is omitted as appropriate. Additionally, in order to facilitate understanding, a configuration that is not actually visible because it is located inside the body part 30 is appropriately indicated by a solid line. Additionally, arrows in Fig. 12 schematically illustrate some of the light beams of the light emitted from the LEDs.

As illustrated in Fig. 12, in the present embodiment, a prism-shaped part 121 is formed on a part of the facing surface of the light guide plate 103. The prism-shaped part 121 according to the present embodiment includes a first prism-shaped part 121A and a second prism-shaped part 121B. In Fig. 12, hatched parts indicate the prism-shaped parts 121, and dotted hatching indicates the prism shape (crest-trough shape).

Light emitted from an LED 106 is diffused and emitted to the outside of the light guide plate 103 through an end of the light guide plate 103. Against this background, by providing the prism-shaped part 121, the light emitted from the LED 106 is prevented from being emitted to the outside of the light guide plate 103, and the light returns to the light guide plate 103.

From this viewpoint, the prism-shaped part 121 is formed in a range excluding the facing surface of the light guide plate 103 located between the LED 106 and a contact region AR. In the present embodiment, the prism-shaped part 121 is formed in the vicinity of ends of the light guide plate 103 where the LED 106 is not provided. Specifically, the first prism-shaped part 121A is formed around the facing surface of the light guide plate 103 facing a third side 111C, and the second prism-shaped part 121B is formed around the facing surface of the light guide plate 103 facing a fourth side 111D. As a result, as schematically illustrated in Fig. 12A, the light emitted from the LED 106 can be prevented from being emitted to the outside through the ends of the light guide plate 103, and the light can be returned to the contact region AR.

Note that the prism-shaped part 121 is preferably formed along a part substantially parallel to a part inclined at a predetermined angle with respect to a reference line set in the light guide plate 103. Fig. 12B is an enlarged view of a part of the second prism-shaped part 121B. A reference line L1 is, for example, a line substantially parallel to each of the third side 111C and the fourth side 111D, in other words, a line substantially parallel to a line connecting a pair of LEDs arranged so as to face each other. As illustrated in Fig. 12B, two points P2 and P3 equidistant from a predetermined position P1 of the reference line L1 are set. A line L2 forming an angle θT1 with respect to the reference line L1 from P2 is set. The second prism-shaped part 121B is formed at a position substantially parallel to the line L2. Additionally, a line L3 forming an angle θT2 (note that relationship of θT1 = θT2 holds) with respect to the reference line L1 from P3 is set. The second prism-shaped part 121B is formed at a position substantially parallel to the line L3. The second prism-shaped part 121B includes prism shapes having different orientations with respect to a line that passes through the position P1 and is substantially orthogonal to the reference line L1.

By forming the second prism-shaped part 121B in this manner, for example, the light emitted from the LED 106D can be effectively returned to the inside of the light guide plate 103 with the prism shape formed on the right side of the second prism-shaped part 121B. Additionally, the light emitted from the LED 106C can be effectively returned to the inside of the light guide plate 103 with the prism shape formed on the left side of the second prism-shaped part 121B. Note that the same applies to the first prism-shaped part 121A.

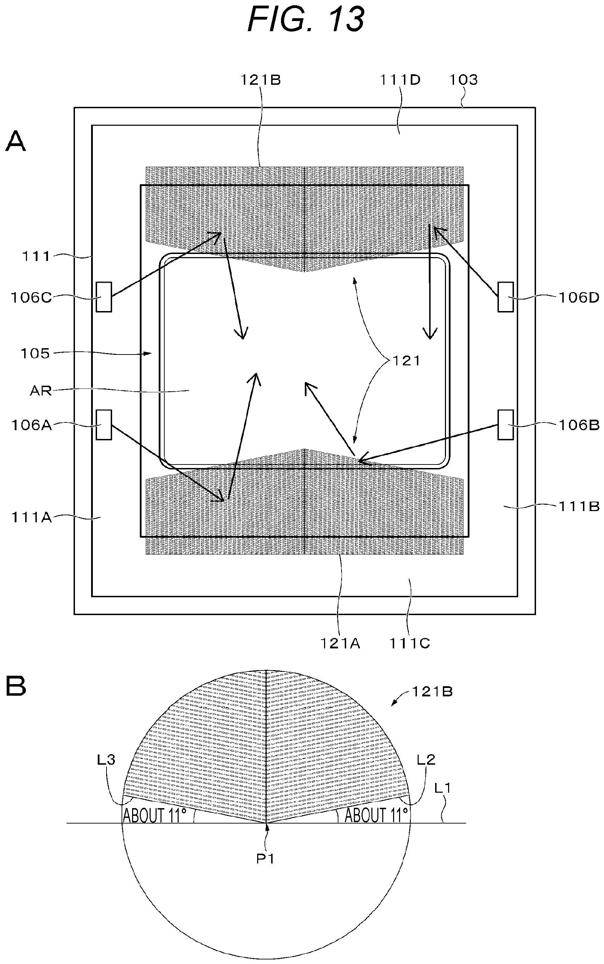

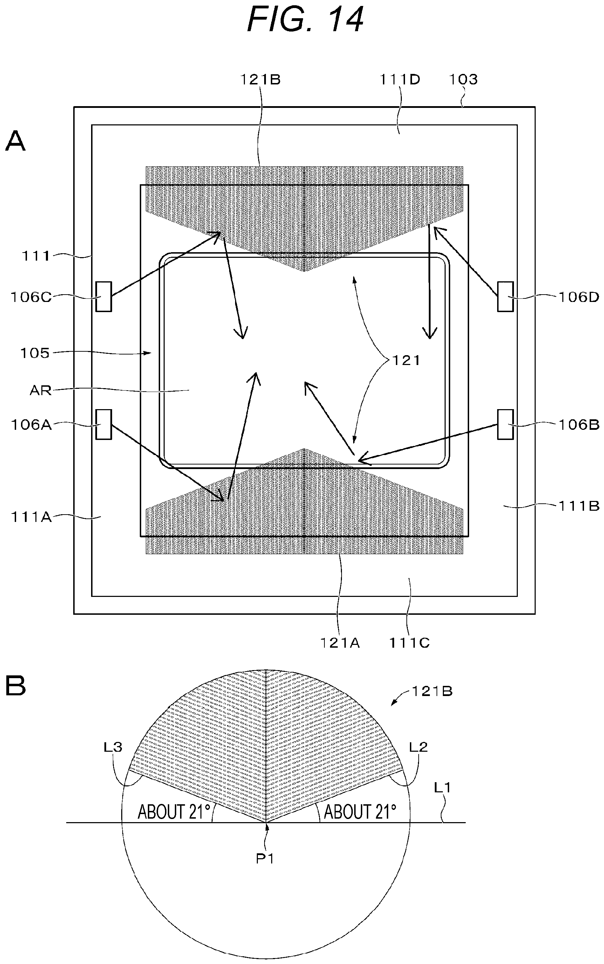

The magnitudes of the angle θT1 and the angle θT2 are set in a range of 10 degrees to 30 degrees, for example. Figs. 13A and 13B illustrate an example in which the magnitudes of the angle θT1 and the angle θT2 are set to 11 degrees. According to the present example, the amount of light to be returned to the inside of the light guide plate 103 can be increased. Additionally, Figs. 14A and 14B illustrate an example in which the magnitudes of the angle θT1 and the angle θT2 are set to 21 degrees. According to the present example, unevenness of light can be reduced. The magnitudes of the angle θT1 and the angle θT2 are appropriately set according to the size of the light guide plate 103, the arrangement position of the LED 106, and the like. Note that Figs. 13 and 14 illustrate examples in which P2 and P3 are not set, and the prism-shaped part 121 is formed along a part substantially parallel to a part inclined at the angle θT1 and the angle θT2 with respect to the reference line L1 from the position P1.

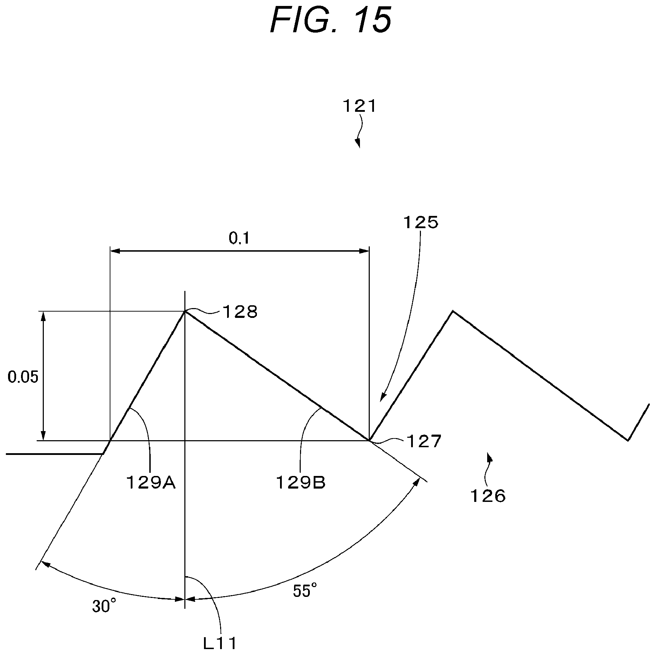

Fig. 15 is a diagram for describing one example of the specific size of the crest-trough shape of the prism-shaped part 121. The prism-shaped part 121 has, for example, a shape in which a plurality of crests 125 and a plurality of troughs 126 are alternately formed. The crest 125 has a top 127 and the trough 126 has a bottom 128. The height of the crest 125 (length from bottom 128 to top 127) is set to about 0.05 mm, for example. Additionally, the width of the trough 126 is set to about 0.1 mm. Additionally, the crest 125 has an inclined surface 129A inclined by about 30 degrees on one side and an inclined surface 129B inclined by 50 degrees to 60 degrees (specifically, 55 degrees) on the other side with respect to a line L11 passing through the bottom 128 and extending in the vertical direction of the light guide plate 103.

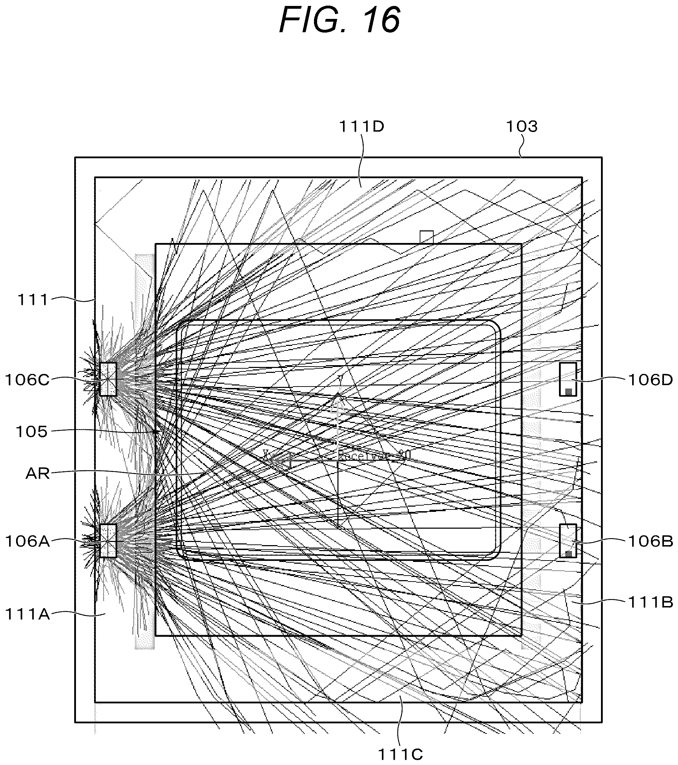

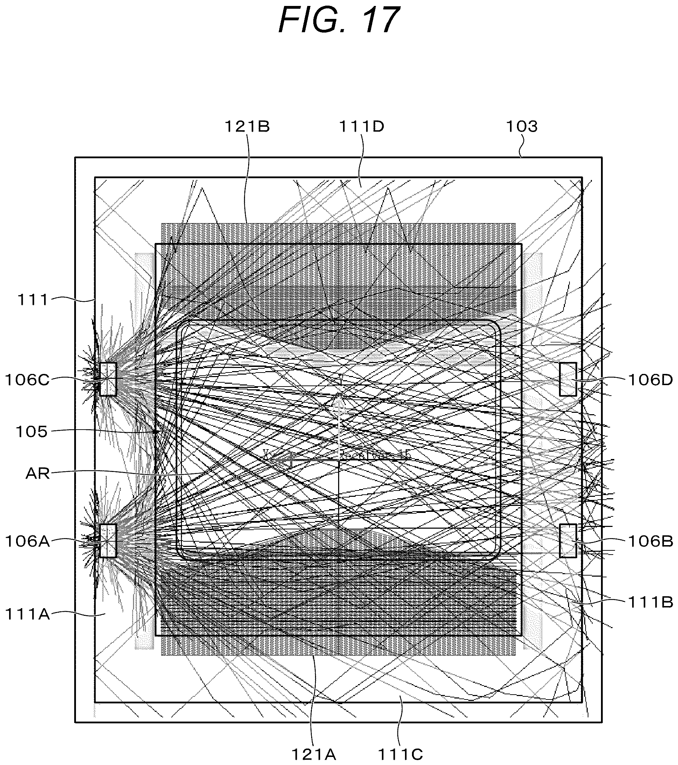

Figs. 16 and 17 are diagrams for describing one example of an effect obtained by forming the prism-shaped part 121. Fig. 16 is a light beam diagram illustrating light beams of light emitted from the LED 106 (LED 106A and LED 106C in this example) in a configuration in which the prism-shaped part 121 is not formed on the facing surface of the light guide plate 103. Fig. 17 is a light beam diagram illustrating light beams of light emitted from the LED 106 (LED 106A and LED 106C in this example) in a configuration in which the prism-shaped part 121 is formed on the facing surface of the light guide plate 103.

As illustrated in Fig. 16, some of the light emitted from the LED 106A and LED 106C is emitted to the outside of the light guide plate 103. On the other hand, as illustrated in Fig. 17, of the light emitted from the LED 106A and the LED 106C, the light directed to the outside of the light guide plate 103 is returned to the inside of the light guide plate 103 by providing the prism-shaped part 121. Accordingly, the loss of the amount of light emitted to the contact region AR can be reduced as much as possible. Note that the same applies to the light emitted from the LED 106B and the LED 106D.

### [Example of manufacturing method of prism-shaped part]

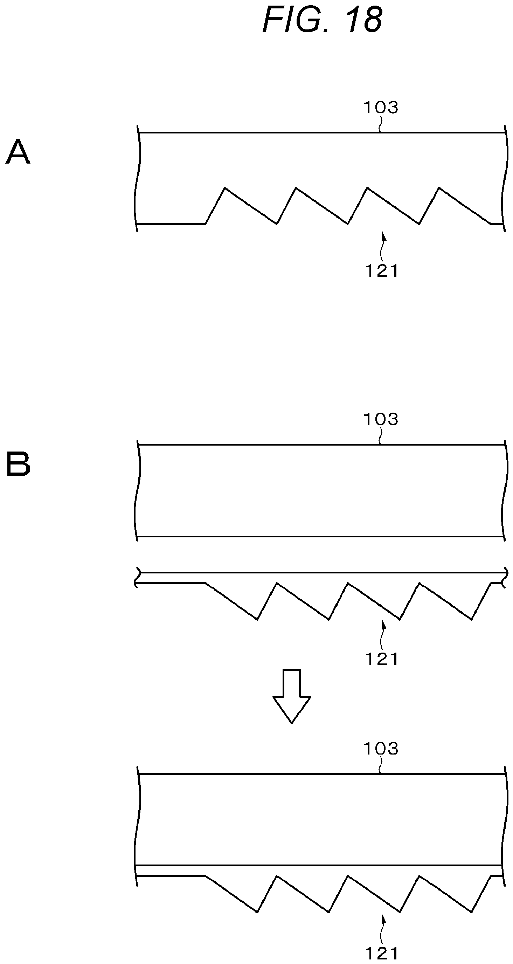

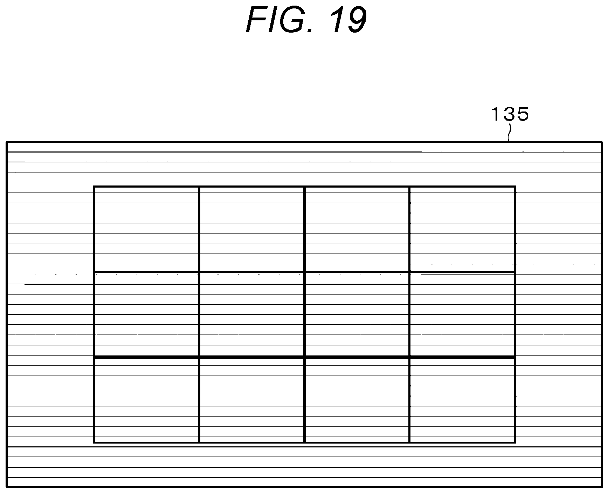

Next, an example of a manufacturing method of the prism-shaped part 121 will be described. As illustrated in Fig. 18A, the prism-shaped part 121 may be formed by performing mechanical processing such as laser processing on the light guide plate 103. Additionally, as illustrated in Fig. 18B, the prism-shaped part 121 formed separately from the light guide plate 103 may be attached to the light guide plate 103 by adhesion or the like to form the prism-shaped part 121. Note that as schematically illustrated in Fig. 19, the prism-shaped part 121 attached to the light guide plate 103 may be formed by making cuts in the sheet-shaped substrate 135.

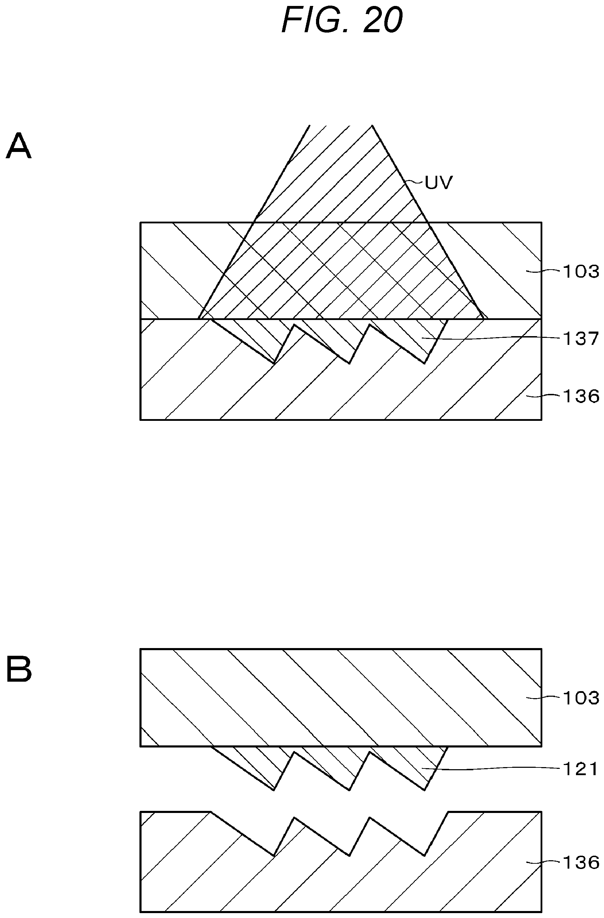

The prism-shaped part 121 may be formed by printing. For example, as illustrated in Fig. 20A, a mold 136 having a pattern corresponding to the prism-shaped part 121 is prepared, and the mold 136 is filled with an ultraviolet curable resin 137 (ink may be used) in a state where the light guide plate 103 and the mold 136 are brought into contact with each other. Thereafter, ultraviolet UV is irradiated. The ultraviolet curable resin 137 is cured by being irradiated with ultraviolet UV. As a result, as illustrated in Fig. 20B, the prism-shaped part 121 is formed on the facing surface of the light guide plate 103.

### <Modification>

Hereinafter, modifications will be described.

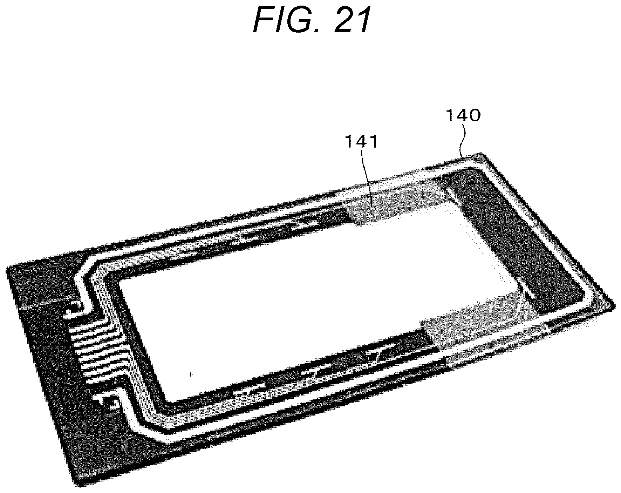

A screen part that conceals the internal structure of the body part 30 may be provided in a peripheral edge part of the front surface of the light guide plate 103 according to the above-described embodiment. Fig. 21 illustrates one example of a screen part 140. A predetermined wiring pattern may be formed on the screen part 140 by printing or the like. As illustrated in Fig. 21, the screen part 140 generally has a frame shape, and is formed by printing black ink. In a case where the screen part 140 is black, the light emitted from the LED 106 is absorbed by the screen part 140, and efficiency may be deteriorated. Against this background, a reflection layer may be provided between the front surface of the light guide plate 103 and the screen part 140. The reflection layer is formed by, for example, printing (mirror printing) a metal layer of nickel, gold, silver, or the like. By providing the reflection layer, the light emitted from the LED 106 can be prevented from being absorbed by the screen part 140. The screen part 140 may be a thin (e.g., about 0.3 mm) metal plate. By applying a metal plate as the screen part 140, the internal structure is not visually recognized to the outside due to transmission of light, and light absorption can be prevented. The metal plate may be subjected to plating processing, hairline processing, coating, or the like.

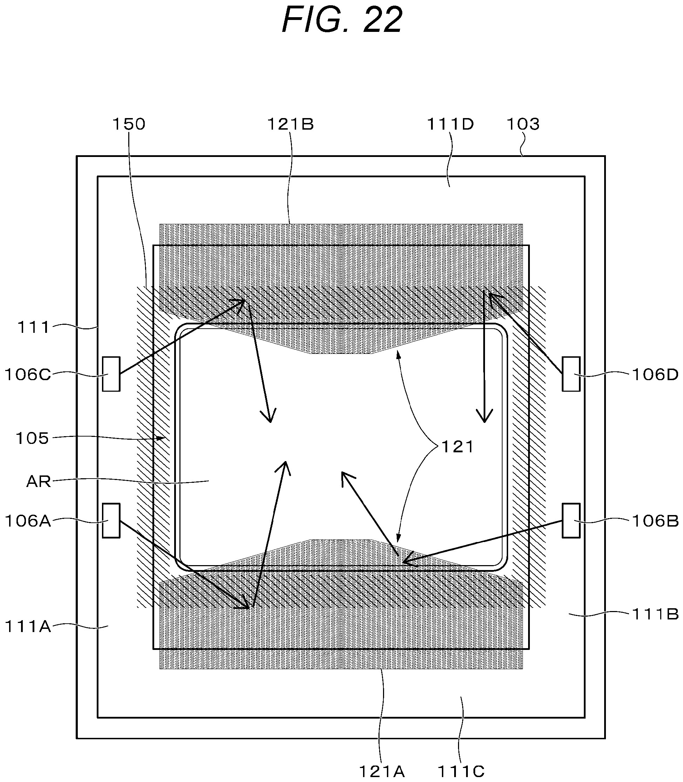

A light diffusion part may be formed on the facing surface of the light guide plate 103 according to the above-described embodiment. For example, as illustrated in Fig. 22, a light diffusion part 150 may be formed in a position opposite to the peripheral position of the contact region AR. The light diffusion part 150 is formed by, for example, mixing a known light diffusion material and printing an optically transparent material. Since the light emitted from the LED 106 can be diffused by forming such a light diffusion part 150, the contrast of the image obtained by the imaging unit 105 can be improved.

In the light guide plate 103 according to the embodiment described above, a shape different from the prism shape, such as an uneven shape, a lenticular shape, or a shape using a plurality of these shapes may be formed. Note that a lenticular shape means that a cross-sectional shape perpendicular to the ridgeline of the protrusion is an arc shape or a substantially arc shape, an elliptic arc shape or a substantially elliptic arc shape, or a parabolic shape or a part of a substantially parabolic shape. Accordingly, a cylindrical shape is also included in the lenticular shape.

An image of a part other than the fingerprint (e.g., palm or the like) may be acquired by the imaging unit 105 described above. Additionally, the number of the LEDs 106 is not limited to four, and can be appropriately set. For example, two LEDs (a total of six LEDs 106) may be disposed on each of the first side 111A, the second side 111B, and the third side 111C. Note, however, that in a case where the electronic device is configured as a wearable device, the number of LEDs 106 is preferably about four from the viewpoint of reducing power consumption. Additionally, while the LED 106 is mounted on the FPC 107 in the above-described embodiments, the LED 106 may be mounted on the same substrate as the substrate on which the image sensor 105A is mounted.

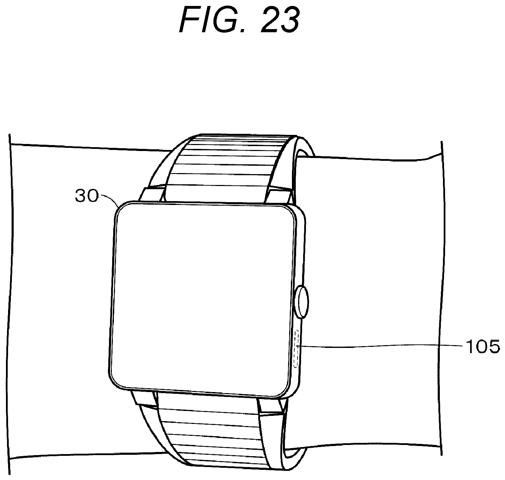

Additionally, as illustrated in Fig. 23, the imaging unit 105, which is one example of a fingerprint sensor, may be disposed so as to image a fingerprint of the fingertip brought into contact with a side surface of the main body part 30.

Alternatively, a protrusion may be disposed on the front surface of the body part 30 in a position (position that wearer desires to touch) appropriate to the sense of the finger, and the user may be guided to the finger position. Additionally, protrusions may be disposed in positions (positions that fingers can easily memorize) appropriate for the user to memorize the sensation on the fingers each time the fingerprint authentication succeeds/fails. The protrusion may be formed integrally with the body part 30 or may be attached to the body part 30.

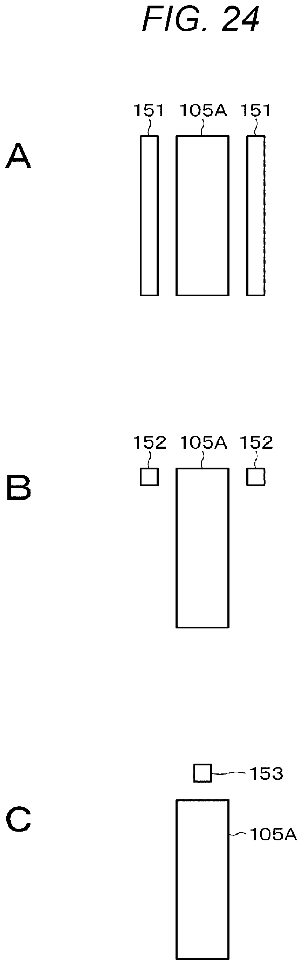

For example, as illustrated in Fig. 24A, protrusions (protrusions 151) having substantially the same length as the sensor (image sensor 105A according to embodiment) are disposed line-symmetrically on both sides of the sensor. Alternatively, as illustrated in Fig. 24B, protrusions (protrusions 152) may be arranged at substantially the same position as the upper end of the sensor and at positions that are line-symmetric on both sides of the sensor. Alternatively, as illustrated in Fig. 24C, a protrusion (protrusion 153) may be disposed on an extension of the sensor. Note that while the protrusion 153 is disposed on the upper side on the extension of the sensor in Fig. 24C, the protrusion 153 may be disposed on the lower side or on both upper and lower sides on the extension of the sensor. These arrangement positions of protrusions can be appropriately combined. Note that in Figs. 24A to 24C, the upper side of the drawing corresponds to the fingertip direction. Instead of the protrusion, a recess, an object including a material having a different texture from the body part 30, an object processed so as to have a different texture from the body part 30, or the like may be disposed.

Note that the contents of the present disclosure should not be interpreted as being limited by the effects exemplified in the present specification.

### REFERENCE SIGNS LIST

- 10: Wristband-type electronic device
- 103: Light guide plate
- 106 (106A to 106D): LED
- 110: Refractive part
- 111: First refractive part
- 111A: First side
- 111B: Second side
- 111C: Third side
- 111D: Fourth side
- 112, 112A, 112B: Second refractive part
- 121: Prism-shaped part
- L1: Reference line
- θT1, θT2: Angle
- 140: Screen part
- 150: Light diffusion part

## Claims

1. An electronic device (10) comprising:
a light guide plate (103);
an imaging unit (105) disposed so as to face the light guide plate (103) and including an image sensor; and
a plurality of light emitting elements (106) disposed around the imaging unit (105); comprising
a refractive part (110) interposed at least between the light emitting element (106) and the light guide plate (103) and refracting light emitted from the light emitting element (106),
**characterized in that**
the refractive part (110) includes a frame-shaped first refractive part (111) and a second refractive part (112, 112A, 112B) integrally formed with the first refractive part (111) and having a parallelogram cross-sectional shape.

2. The electronic device (10) according to claim 1, wherein
the first refractive part (111) includes a first side (111A) and a second side (111B) facing each other, and
the second refractive part (112, 112A, 112B) is formed on a front surface of each of the first side (111A) and the second side (111B).

3. The electronic device (10) according to claim 2, wherein
the light emitting element (106) is disposed on an opposite side of the second refractive part (112, 112A, 112B) formed on each of the first side (111A) and the second side (111B).

4. The electronic device (10) according to claim 3, wherein
two light emitting elements (106) are disposed on the opposite side of each of the second refractive parts (112A, 112B).

5. The electronic device (10) according to claim 1, wherein
a magnitude of each of a pair of diagonal angles in the cross section of the second refractive part (112, 112A, 112B) is set within a range of 40 degrees to 45 degrees.

6. The electronic device (10) according to claim 2, wherein
the light guide plate (103) includes a facing surface that is a surface on a side facing the imaging unit (105), and
a prism-shaped part is formed in a part of the facing surface.

7. The electronic device (10) according to claim 6, wherein
the first refractive part (111) includes a third side (111C) and a fourth side (111D) facing each other, and
the prism-shaped part is formed around a part facing each of the third side (111C) and the fourth side (111D) on the facing surface of the light guide plate (103).

8. The electronic device (10) according to claim 7, wherein
the prism-shaped part is formed along a part substantially parallel to a part inclined at a predetermined angle with respect to a reference line set in the light guide plate (103).

9. The electronic device (10) according to claim 8, wherein
the reference line is a line substantially parallel to each of the third side and the fourth side.

10. The electronic device (10) according to claim 8, wherein
the predetermined angle is set within a range of 10 degrees to 30 degrees.

11. The electronic device (10) according to claim 1, wherein
the light guide plate (103) includes a facing surface that is a surface on a side facing the imaging unit (105), and
an uneven shape or a lenticular shape is formed in a part of the facing surface.

12. The electronic device (10) according to claim 1, wherein
the light guide plate (103) includes a front surface that is a surface opposite to a surface facing the imaging unit (105),
a screen part (140) is formed in a peripheral edge part of the front surface, and
a reflection layer is formed between the front surface and the screen part.

13. The electronic device (10) according to claim 1, wherein
the light guide plate (103) includes a facing surface that is a surface on a side facing the imaging unit (105), and
a light diffusion part (150) is formed on the facing surface.

14. The electronic device (10) according to claim 1 configured as a wearable device.

## Patentansprüche

1. Elektronische Vorrichtung (10), umfassend:
eine Lichtleiterplatte (103);
eine Bildgebungseinheit (105), die so angeordnet ist, dass sie zu der Lichtleiterplatte (103) weist und einen Bildsensor einschließt; und
eine Vielzahl von Licht emittierenden Elementen (106), die um die Bildgebungseinheit (105) herum angeordnet sind; umfassend
ein refraktives Teil (110), das mindestens zwischen das Licht emittierende Element (106) und die Lichtleiterplatte (103) geschoben ist und Licht bricht, das von dem Licht emittierenden Element (106) emittiert wird, **dadurch gekennzeichnet, dass** das refraktive Teil (110) ein rahmenförmiges erstes refraktives Teil (111) und ein zweites refraktives Teil (112, 112A, 112B) einschließt, das integral mit dem ersten refraktiven Teil (111) gebildet ist und einen parallelogrammförmigen Querschnitt hat.

2. Elektronische Vorrichtung (10) nach Anspruch 1, wobei das erste refraktive Teil (111) eine erste Seite (111A) und eine zweite Seite (111B) einschließt, die zueinander weisen, und
das zweite refraktive Teil (112, 112A, 112B) auf einer vorderen Oberfläche von jeder von der ersten Seite (111A) und der zweiten Seite (111B) gebildet ist.

3. Elektronische Vorrichtung (10) nach Anspruch 2, wobei das Licht emittierende Element (106) auf einer entgegengesetzten Seite des zweiten refraktiven Teils (112, 112A, 112B) angeordnet ist, das auf jeder von der ersten Seite (111A) und der zweiten Seite (111B) gebildet ist.

4. Elektronische Vorrichtung (10) nach Anspruch 3, wobei zwei Licht emittierende Elemente (106) auf der entgegengesetzten Seite von jedem der zweiten refraktiven Teile (112A, 112B) angeordnet sind.

5. Elektronische Vorrichtung (10) nach Anspruch 1, wobei eine Größe von jedem von einem Paar diagonaler Winkel im Querschnitt des zweiten refraktiven Teils (112, 112A, 112B) auf innerhalb eines Bereichs von 40 Grad bis 45 Grad festgelegt ist.

6. Elektronische Vorrichtung (10) nach Anspruch 2, wobei
die Lichtleiterplatte (103) eine weisende Oberfläche einschließt, die eine Oberfläche auf einer Seite ist, die zu der Bildgebungseinheit (105) weist, und
ein prismenfärmiges Teil in einem Teil der weisenden Oberfläche gebildet ist.

7. Elektronische Vorrichtung (10) nach Anspruch 6, wobei
das erste refraktive Teil (111) eine dritte Seite (111C) und eine vierte Seite (111D) einschließt, die zueinander weisen, und
das prismenförmige Teil um ein Teil herum gebildet ist, das zu jeder von der dritten Seite (111C) und der vierten Seite (111D) der weisenden Oberfläche der Lichtleiterplatte (103) weist.

8. Elektronische Vorrichtung (10) nach Anspruch 7, wobei das prismenförmige Teil entlang eines Teils gebildet ist, das im Wesentlichen parallel zu einem Teil ist, das in einem vorbestimmten Winkel in Bezug zu einer Referenzlinie geneigt ist, die in der Lichtleiterplatte (103) festgelegt worden ist.

9. Elektronische Vorrichtung (10) nach Anspruch 8, wobei die Referenzlinie eine Linie ist, die im Wesentlichen parallel zu jeder von der dritten Seite und der vierten Seite ist.

10. Elektronische Vorrichtung (10) nach Anspruch 8, wobei der vorbestimmte Winkel auf innerhalb eines Bereichs von 10 Grad bis 30 Grad festgelegt ist.

11. Elektronische Vorrichtung (10) nach Anspruch 1, wobei
die Lichtleiterplatte (103) eine weisende Oberfläche einschließt, die eine Oberfläche auf einer Seite ist, die zu der Bildgebungseinheit (105) weist, und
eine ungleichförmige Formgebung oder eine linsenförmige Formgebung in einem Teil der weisenden Oberfläche gebildet ist.

12. Elektronische Vorrichtung (10) nach Anspruch 1, wobei
die Lichtleiterplatte (103) eine vordere Oberfläche einschließt, die eine Oberfläche entgegengesetzt zu einer Oberfläche ist, die zu der Bildgebungseinheit (105) weist,
ein Bildschirmteil (140) in einem peripheren Randteil der vorderen Oberfläche gebildet ist, und eine Reflexionsschicht zwischen der vorderen Oberfläche und dem Bildschirmteil gebildet ist.

13. Elektronische Vorrichtung (10) nach Anspruch 1, wobei
die Lichtleiterplatte (103) eine weisende Oberfläche einschließt, die eine Oberfläche auf einer Seite ist, die zu der Bildgebungseinheit (105) weist, und
ein Lichtdiffusionsteil (150) auf der weisenden Oberfläche gebildet ist.

14. Elektronische Vorrichtung (10) nach Anspruch 1, die als tragbare Vorrichtung ("Wearable") ausgestaltet ist.

## Revendications

1. Dispositif électronique (10) comprenant :
une plaque de guidage de la lumière (103) ;
une unité d'imagerie (105) disposée de manière à faire face à la plaque de guidage de la lumière (103) et comportant un capteur d'images ; et
une pluralité d'éléments émetteurs de lumière (106) disposés autour de l'unité d'imagerie (105) ; comprenant
une partie réfractive (110) intercalée au moins entre l'élément émetteur de lumière (106) et la plaque de guidage de la lumière (103) et réfractant la lumière émise depuis l'élément émetteur de lumière (106),
**caractérisé en ce que**
la partie réfractive (110) comporte une première partie réfractive en forme de cadre (111) et une deuxième partie réfractive (112, 112A, 112B) formée d'une seule pièce avec la première partie réfractive (111) et ayant pour forme de section transversale un parallélogramme.

2. Dispositif électronique (10) selon la revendication 1, dans lequel
la première partie réfractive (111) comporte un premier côté (111A) et un deuxième côté (111B) se faisant face, et
la deuxième partie réfractive (112, 112A, 112B) est formée sur une surface avant à la fois du premier côté (111A) et du deuxième côté (111B).

3. Dispositif électronique (10) selon la revendication 2, dans lequel
l'élément émetteur de lumière (106) est disposé sur un côté opposé de la deuxième partie réfractive (112, 112A, 112B) formé à la fois sur le premier côté (111A) et le deuxième côté (111B).

4. Dispositif électronique (10) selon la revendication 3, dans lequel
deux éléments émetteurs de lumière (106) sont disposés sur le côté opposé de chacune des deuxièmes parties réfractives (112A, 112B).

5. Dispositif électronique (10) selon la revendication 1, dans lequel
une amplitude de chaque angle d'une paire d'angles de diagonale dans la section transversale de la deuxième partie réfractive (112, 112A, 112B) est définie dans une plage de 40 degrés à 45 degrés.

6. Dispositif électronique (10) selon la revendication 2, dans lequel
la plaque de guidage de la lumière (103) comporte une surface en opposition qui est une surface sur un côté faisant face à l'unité d'imagerie (105), et
une partie en forme de prisme est formée dans une partie de la surface en opposition.

7. Dispositif électronique (10) selon la revendication 6, dans lequel
la première partie réfractive (111) comporte un troisième côté (111C) et un quatrième côté (111D) se faisant face, et
la partie en forme de prisme est formée autour d'une partie faisant face à la fois au troisième côté (111C) et au quatrième côté (111D) sur la surface en opposition de la plaque de guidage de la lumière (103).

8. Dispositif électronique (10) selon la revendication 7, dans lequel
la partie en forme de prisme est formée le long d'une partie sensiblement parallèle à une partie inclinée à un angle prédéterminé par rapport à une ligne de référence définie dans la plaque de guidage de la lumière (103).

9. Dispositif électronique (10) selon la revendication 8, dans lequel
la ligne de référence est une ligne sensiblement parallèle à la fois au troisième côté et au quatrième côté.

10. Dispositif électronique (10) selon la revendication 8, dans lequel
l'angle prédéterminé est défini dans une plage de 10 degrés à 30 degrés.

11. Dispositif électronique (10) selon la revendication 1, dans lequel
la plaque de guidage de la lumière (103) comporte une surface en opposition qui est une surface sur un côté faisant face à l'unité d'imagerie (105), et
une forme irrégulière ou une forme lenticulaire est formée dans une partie de la surface en opposition.

12. Dispositif électronique (10) selon la revendication 1, dans lequel
la plaque de guidage de la lumière (103) comporte une surface avant qui est une surface à l'opposé d'une surface faisant face à l'unité d'imagerie (105),
une partie faisant écran (140) est formée dans une partie de bord périphérique de la surface avant, et
une couche de réflexion est formée entre la surface avant et la partie faisant écran.

13. Dispositif électronique (10) selon la revendication 1, dans lequel
la plaque de guidage de la lumière (103) comporte une surface en opposition qui est une surface sur un côté faisant face à l'unité d'imagerie (105), et
une partie de diffusion de la lumière (150) est formée sur la surface en opposition.

14. Dispositif électronique (10) selon la revendication 1 configuré comme un dispositif portable.
